# EUROPÄISCHE PATENTSCHRIFT

(11) **EP 2 603 192 B1**
(45) Veröffentlichungstag und Bekanntmachung des Hinweises auf die Patenterteilung: **27.02.2019**
(21) Anmeldenummer: 11752102.1
(22) Anmeldetag: 04.08.2011
(51) Int. Cl.: A61K 6/00, A61K 6/083

(54) **VERWENDUNG VON KIESELSÄURE(N) ZUR EIGENSCHAFTSVERBESSERUNG VON DENTALMATERIAL UND ENTSPRECHENDE VERFAHREN**
USE OF SILICIC ACID(S) FOR IMPROVING THE PROPERTIES OF DENTAL MATERIALS AND CORRESPONDING METHOD
UTILISATION D'ACIDE(S) SILICIQUE(S) POUR AMÉLIORER LES PROPRIÉTÉS DE MATÉRIAUX DENTAIRES ET PROCÉDÉS CORRESPONDANTS

(30) Priorität: 12.08.2010 DE 102010034194
(43) Veröffentlichungstag der Anmeldung: 19.06.2013
(73) Patentinhaber: Kulzer GmbH, 63450 Hanau (DE)
(72) Erfinder: RUPPERT, Klaus, 63477 Maintal (DE); HOHMANN, Alfred, 61389 Schmitten (DE); SCHNACK, Alexander, 63879 Weibersbrunn (DE)
(74) Vertreter: Bendele, Tanja
(86) Internationale Anmeldenummer: PCT/EP2011/003902
(87) Internationale Veröffentlichungsnummer: WO 2012/019735

(56) Entgegenhaltungen:
- WO-A1-03/055802
- GB-A- 2 020 197
- US-A- 4 157 387
- US-A- 4 267 097
- US-A- 4 707 504
- US-A1- 2003 181 541

## Beschreibung

Die Erfindung betrifft die Verwendung bestimmter Kieselsäure(n) zur Einstellung der Gebrauchseigenschaften von Dentalmaterial sowie entsprechende Verfahren.

Komposite für Dentalanwendungen (Füllungskomposite, Verblendkomposite) bestehen typischerweise aus einer Monomermischung, Initiatoren, Stabilisatoren, Füllstoffen und Rheologiemodifizierern. Obwohl Rheologiemodifizierer nur zu einem geringen Anteil enthalten sind, werden hierdurch wichtige Handlings- und damit Produkteigenschaften bestimmt. Die Handlingseigenschaften drücken sich beispielsweise in der Modellierfähigkeit, der Standfestigkeit und einem sogenannten "Wackelpuddingeffekt" aus, der weiter unten bei den Beispielen beschrieben ist. Sie beruhen beispielsweise auf Viskosität, Oberflächeneigenschaften, Elastizität und Thixotropiegrad. Ein weiterer möglicher Faktor sind Lagereigenschaften und Mischeigenschaften, d.h. die Eignung zum Weiterverarbeiten durch Anmischen.

Die meisten Rheologiemodifizierer bestehen aus reinem SiO₂ und weisen damit eine Brechzahl von ∼ 1,46 auf. Diese Produkte weisen oft einen Primärpartikeldurchmesser im Nanometerbereich auf, sind aber aufgrund des Herstellungsprozesses agglomeriert und haben Agglomeratgrößen von einigen 100 nm.

Damit ergibt sich eine erhebliche Differenz zwischen der Brechzahl der Dentalgläser sowie der üblichen Monomermischungen einerseits und dem Rheologiemodifizierer andererseits. Die Brechzahldifferenz führt zu einer deutlichen Abnahme der Transparenz und damit zu einer Verschlechterung der ästhetischen Eigenschaften.

In US 4,707,504 (EP 0 172 513 A2) wird die Verwendung poröser Füllstoffe mit relativ großem mittleren Partikeldurchmesser von 2-12-µm, Porendurchmesser von 10-50 nm und spezifischer Oberfläche bis 600 m²/g zur Verbesserung von Glanz und Polierbarkeit von Dentalwerkstoffen empfohlen.
Gegenstand der Erfindung ist die Verwendung mikroporöser anorganischer Füllstoffe in polymerisierbaren Dentalmassen, dadurch gekennzeichnet, daß die Füllstoffe a) eine mittlere Teilchengröße von 0,5 - 50 µm, vorzugsweise 1-20 µm; b) eine BET-Oberfläche von mindestens 200 m²/g, vorzugsweise 300-600 m²/g; c) ein Porenvolumen von 0,7-5 ml/g, vorzugsweise 1-3 ml/g; und d) einen Porendurchmesser von 10-50 nm, vorzugsweise ca. 20 nm, aufweisen.

Von spezifischen Oberflächen über 600 m²/g wird dort wegen der zu hohen Opazität abgeraten (Vergleichsbeispiel 750 m²/g).

Im Vorliegenden stellt sich die Aufgabe, die Gebrauchseigenschaften von Dentalkompositen im ungehärteten Zustand günstig zu beeinflussen, ohne die Produkteigenschaften - besonders die optischen und mechanischen des fertig gehärteten Materials - zu verschlechtern.

Die Aufgabe wird erfindungsgemäß durch Einsatz von Kieselsäure mit hoher spezifischer Oberfläche, innerer Porenstruktur, hydrophobierter Oberfläche sowie einer nur schwachen Agglomeration der Primärpartikel gelöst. Dafür eignet sich beispielsweise Kieselsäure vom Typ Nanogel TLD 201 der Fa. Cabot. Dabei handelt es sich um ein Aerogel mit Partikelgrößen im Bereich von 5 - 15 µm und Porendurchmessern von etwa 20 nm (WO 2006102568 A2, Absatz [39]).

Die Erfindung betrifft demnach die Verwendung bestimmter Kieselsäuren zur Einstellung der Gebrauchseigenschaften von Dentalmaterial, wobei die Kieselsäure hochdispers ist, einen Porendurchmesser von 15 - 30 nm, eine innere Porenstruktur, eine Korngröße von d₅₀ 7 - 10 µm und eine hydrophobe Oberflächenmodifizierung durch Oberflächenbelegung mit Trimethylsilylgruppen aufweist, und die Kieselsäure als Rheologiemodifizierer in einem Verblendkomposit oder einem Füllungskomposit oder einem Prothesenwerkstoff oder einem Material zur Herstellung künstlicher Zähne oder einem Dentalzement oder einem Abformmaterial oder einem Pastenopaker eingesetzt wird.. Weitere und bevorzugte Ausführungsformen sind den Ansprüchen 2 - 8 zu entnehmen.

Wobei die Kieselsäure aufgrund ihrer Porosität zu 95% aus Luft besteht kann.

Wobei die hochdisperse Kieselsäure eine spezifische Oberfläche von > 700 m²/g aufweisen kann.

Die Erfindung betrifft auch ein Verfahren zur Einstellung der Gebrauchseigenschaften von Dentalmaterial
mit den Schritten:
**A** Herstellen einer Vor-Mischung aus mindestens Dentalmonomer(en), Initiator(en) und Kieselsäure mit innerer Porenstruktur mit Porendurchmesser von 15 - 30 nm und einer hydrophoben Oberflächenmodifizierung durch Oberflächenbelegung mit Trimethylsilylgruppen;
**B** Behandeln der Vormischung mit einem Mischgerät,
**C** Nachbehanden der entstanden Mischung mit starken Scherkräften. Sowie ein Verfahren gemäß dem Anspruch 10, wobei Schritt **B** mit einem Rotationsmischgerät durchgeführt wird und gemäß Anspruch 11, wobei Schritt **C** mit einem Dreiwalzenstuhl, einem Rotor- Stator-Mischer oder Ultraschalleinwirkung durchgeführt wird..

Unter innerer Porenstruktur ist ein untereinander verbundenes Kanalsystem zu verstehen. Die Porendurchmesser betragen zweckmäßig 5-30 nm, vorzugsweise um 20 nm.
Geeignete Kieselsäuren haben mittlere Partikelgrößen von 5 bis 1200 µm, insbesondere 5 bis 500 µm und bevorzugt 5 - 15 µm. Es gibt aber auch gleich poröse Kieselsäurequalitäten mit 0,5 bis 4 mm Partikelgröße. Wobei durchschnittliche Primärpartikelgröße von < 100 nm besitzen können. Die hochdisperse Kieselsäure aus agglomerierten Primärpartikeln mit einem überwiegenden Partikeldurchmesser von 20 - 50 nm bestehen kann. Die hochdisperse Kieselsäure aus agglomerierten Primärpartikeln mit einem überwiegenden Partikeldurchmesser von 20 - 40 nm bestehen kann. Wobei die agglomerierten Partikel der hochdispersen Kieselsäure durch hohe Scherkräfte, insbesondere Drei-Walzenstuhl, in die Primärpartikel aufgespalten werden können.

Die Kieselsäuren sind in der Regel und auch vorzugsweise hydrophobiert, für gewöhnlich durch Silanisierung, bei der die Oberfläche mit -O-SiR₃ Gruppen versehen wird, worin R gleiche oder verschiedene einwertige, gegebenenfalls substituierte Kohlenwasserstoffreste bedeutet, wie etwa in EP379785B2 beschrieben.

### Geeignete Dentalmaterialien können folgende Komponenten enthalten:

### Füllstoffe

### Dentalgläser:

Als Dentalgläser eignen sich besonders Bariumglaspulver und/oder Strontiumglaspulver. Die mittlere Partikelgröße der grobteiligen Dentalgläser beträgt vorzugsweise 5 - 10 µm, insbesondere um 7 µm und die der feinteiligen 0,5 - 2 µm, insbesondere 1 µm. Optional vorhandene weitere Dentalgläser haben z.B. mittlere Korngrößen von 2 - 5 oder 10 - 50 µm.

Die Füllstoffkomponente kann demnach Dentalgläser mit insgesamt drei oder mehr Kornfraktionen aufweisen.

Weitere, herkömmliche, auf dem Dentalgebiet übliche Füllstoffe, wie etwa Quarz-, Glaskeramik- oder Mischungen davon. Darüber hinaus können die Komposite Füllstoffe zur Erzielung einer erhöhten Röntgenopazität enthalten. Die mittlere Partikelgröße des röntgenopaken Füllstoffs liegt vorzugsweise im Bereich von 100 - 300 nm, insbesondere 180 - 300 nm. Als röntgenopake Füllstoffe eignen sich z.B. die in der DE 35 02 594 A1 beschriebenen Fluoride der Seltenen Erdmetalle, d. h. die Trifluoride der Elemente 57 - 71. Ein besonders bevorzugt verwendeter Füllstoff ist Ytterbiumfluorid, insbesondere Ytterbiumtrifluorid mit einer mittleren Partikelgröße von etwa 300 nm. Die Menge des röntgenopaken Füllstoffs beträgt vorzugsweise 10 - 50 Gew.-%, besonders bevorzugt 20 - 30 Gew.-%, bezogen auf den Gesamtfüllstoffgehalt.

Außerdem können gefällte Mischoxide, wie beispielsweise ZrO₂/SiO₂ als Füllstoffe eingesetzt werden. Bevorzugt sind Mischoxide mit einer Partikelgröße von 200 - 300 nm und insbesondere etwa 200 nm. Die Mischoxidpartikel sind vorzugsweise kugelförmig und weisen eine einheitliche Größe auf. Die Mischoxide haben vorzugsweise einen Brechungsindex von 1,52 - 1,55. Gefällte Mischoxide werden vorzugsweise in Mengen 25 - 75 Gew.-% und besonders 40 - 75 Gew.-% verwendet.

Die Füllstoffe sind zur Verbesserung der Haftung zwischen Füllstoff und organischer Matrix bevorzugt silanisiert. Als Haftvermittler eignet sich besonders alpha - Methacryloxypropyltrimethoxysilan. Die Menge des eingesetzten Haftvermittlers richtet sich nach der Art und BET-Oberfläche des Füllstoffs.

### Dentalmonomere:

Beispiele geeigneter Monomere sind die im Dentalbereich üblichen, wie z.B. monomere (Meth)acrylate wie Ethyleneglycoldimethacrylat EDMA, Diethylenglycoldimethacrylat, Triethylenglycoldimethacrylate TEGDMA, Glyceroldimethacrylat GDMA, Glyceroltrimethacrylate, Trimethylolpropantrimethacrylat, Pentaerythritoldimethacrylat, Pentaerythritoltrimeth-acrylat, Pentaerythritoltetramethacrylat, Derivate von Bisphenol A wie Bisphenol-A-dimeth-acrylat und Bisphenol-A-diglycoldimethacrylat, Urethanmethacrylat erhältlich aus Diisocyanaten und Hydroxyalkylmethacrylaten, sowie Reaktionsprodukte von Polyolen, Diisocyanaten und Hydroxyalkylmethacrylaten gemäß DE 37 03 080 A1 oder DE 37 03 120 A1; C1-12-, vorzugsweise C1-4-Alkylmethacrylate wie Methylmethacrylat, Ethylmethacrylat, n-Propylmethacrylat, Isopropylmethacrylat, n-Butylmethacrylat and t-Butylmethacrylat, Hydroxyalkyl-C1-4-methacrylate wie 2-Hydroxyethylmethacrylat, 2-Hydroxypropylmethacrylat, Diethyleneglycolmonomethacrylat, Triethylenglycolmonomethacrylat, Alkoxy-C1-4 alkylmethacrylate wie 2-Methoxyethylmethacrylat, 3-Methoxybutylmethacrylat und Ethyltriglycolmethacrylat. Geeignete Monomere sind davon jeweils die Monomere selbst, daraus hergestellte polymerisierbare Präpolymere sowie Mischungen von diesen.

Einige der Monomere fungieren als Vernetzer: Als multifunktionelle Vernetzer kommen außer TEGDMA und UDMA in Frage: Diethylenglycoldi(meth)acrylat, Decandioldi(meth)acrylat, Trimethylolpropantri(meth)acrylat, Pentaerythrittetra(meth)acrylat sowie Butandioldi(meth)acrylat, 1,10-Decandioldi(meth)acrylat, 1,12-Dodecandioldi(meth)acrylat.

Zur Initiierung der Polymerisation enthalten die Komposite gewöhnlich mindestens einen Polymerisationsinitiator, beispielsweise einen Initiator für die radikalische Polymerisation. Je nach Art des verwendeten Initiators können die Mischungen kalt, durch Licht oder heiß polymerisierbar sein.

Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch alpha, alpha '-Azo-bis(isobutyroethylester), Benzpinakol und 2,2'-Dimethylbenzpinakol sind geeignet.

Als Photoinitatoren kommen z.B. Benzoinalkylether oder -ester, Benzilmonoketale, Acylphosphinoxide oder aliphatische und aromatische 1.2-Diketoverbindungen, wie beispielsweise 2,2-Diethoxyacetophenon 9,10-Phenanthrenchinon, Diacetyl, Furil, Anisil, 4,4'-Dichlorbenzil und 4,4'-Dialkoxybenzil oder Campherchinon, in Frage. Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Heißhärtung eingesetzt werden.

Als Initiatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet.

Es können auch dual härtende Systeme verwendet werden, z. B. Photoinitiatoren mit Aminen und Peroxiden.

Die Initiatoren werden vorzugsweise in Mengen von 0,01 bis 1 Gew.-% bezogen auf die Gesamtmasse der Mischung verwendet.

Bei der Kaltpolymerisation kann es zweckmäßig sein, wenn das Kompositmaterial aufgeteilt in zwei Komponenten vorliegt, die zur Aushärtung durch Vermischen vorgesehen sind. Es ist auch möglich, das Material so bereitzustellen, dass es sowohl durch Licht als auch durch Vermischen zweier Komponenten zu härten ist.

### Pigmente:

Üblicherweise werden Dentalfüllungskomposite in verschiedenen Zahnfarben eingefärbt. Die dafür üblichen Pigmente sind dem Fachmann geläufig.

### Hilfsstoffe:

Hier kommen neben den erfindungsgemäßen Kieselsäuren, die als Thixotropie und Strukturverbesserer dienen, in Frage: Stabilisatoren, UV-Absorber, Farbstoffe und/oder Gleitmittel. Die Hilfsstoffe werden ggf. in einer Menge von jeweils bis zu etwa 0,5 Gew.-% eingesetzt.

Geeignete Stabilisatoren sind z. B. Hydrochinonmonomethylether oder 2,6-Di-tert.-butyl-4-methylphenol (BHT).

Die Mischungen werden vorzugsweise durch Bereitstellen einer Vor-Mischung der Monomere mit der Kieselsäure hergestellt. Intensives Vermischen mit geeigneten Mischapparaturen und gegebenenfalls anschließende Behandlung in einer Dreiwalze, einem Rotor-Stator-Mischer oder einer Ultraschall-Mischapparatur ergeben die gewünschten homogenenen Qualitäten.

Die Erfindung wird durch das folgende Beispiel näher erläutert. Teile und Prozentangaben beziehen sich wie in der übrigen Beschreibung auf das Gewicht, sofern nicht anders angegeben.

### Ausführungsbeispiele:

### Beispiel 1: Wirkung verschiedener Rheologiemodifizierer

Aus 71 % Urethandimethacrylat, 17 % Dodecandioldimethacrylat, 2 % Campherchinon und 10 % SiO₂ verschiedener Qualität gemäß nachfolgender Tabelle I werden Vor-Mischungen hergestellt. Die jeweilige Vor-Mischung wird ca. 3 min bei ca. 1500 min⁻¹ in einem Rotationsmischer Speedmixer® DAC 150 FVZ (Firma Hauschild, Drehzahl variabel von 500 bis 3500 rpm) gemischt. Zur besseren Homogenisierung wird die erhaltene Monomer-Kieselsäuremischung anschließend über eine Dreiwalze *EXAKT (EXAKT-Apparatebau)* gegeben. Der SiO₂-Anteil liegt bei diesem Beispiel nur bei 10 Gew. %, damit auch stark verdickende Rheologiemodifizierer in ihren Eigenschaften beurteilt werden können. Es ist selbstverständlich möglich, Mischungen mit wesentlich höherem SiO₂-Anteil in gleicher Weise herzustellen und zu behandeln.

| Tabelle I | **b-Wert** | **Transparenz** | **Biegefestigkeit** | **E-Modul** | **Konsistenz [1]** |
|---|---|---|---|---|---|
| **Füllstoff** | | **[%]** | **[MPa]** | **[MPa]** | |
| Sipernat 160 (Evonik) [2] | 18,29 | 83,08 | 86 | 1884 | 3 |
| Aerosil OX 50 sil. < 160 µm | 19,12 | 83,8 | 67 | 1937 | 2 |
| Aerosil OX 50 40 nm | 25,38 | 74,59 | 86 | 1906 | 4 |
| Neomat 70 nm | 20,73 | 65,08 | 72 | 1792 | 2 |
| Sipernat 350 (Evonik) [3] | 31,61 | 60,91 | 88 | 1900 | 3 |
| Aerosil 130 | 19,40 | 87,59 | 83 | 1934 | 4 |
| Aerosil R972 [4] | 17,93 | 89,84 | 90 | 1957 | 3 |
| Sident 10 | 28,21 | 59,58 | 78 | 1901 | 2 |
| Sipernat 570 | 17,79 | 84,79 | 86 | 1966 | 3 |
| Nanogel TLD 201 | 10,98 | 94,82 | 81 | 1738 | 5 |
| Sipernat D10 | 30,99 | 76,89 | 92 | 2014 | 3 |
| Aerosil R812S[5] | 16,68 | 93,42 | 79 | 1841 | 3 |
| Aerosil OX 50 [6] sil. < 25 µm | 23,41 | 80,43 | 85 | 1852 | 2 |
| Aerosil OX 50 sil. < 50 µm | 25,22 | 80,57 | 86 | 2020 | 2 |
| Aerosil OX 50 sil. < 100 µm | 25,27 | 81,03 | 77 | 2074 | 2 |
| Aerosil COK 84 [7] | 17,32 | 81,34 | 92 | 2031 | 4 |
| Sipernat 500 LS[8] | 18,63 | 81,44 | 91 | 2120 | 3 |

| | | | | | |
|---|---|---|---|---|---|
| *[1] Bewertung der Konsistenz nach Schulnoten (subjektiv): 1 = niedrigviskos (wasserähnlich); 3 = gelartig (Wackelpudding); 6 = hochviskos*/*pastös* [2] spez. Oberfläche 165 m²/g, Mittlere Teilchengröße (ASTM C690) 7 µm Teilchengröße D50 (ISO 13320-1) 11 µm [3] spez. Oberfläche 50 m²/g, Mittlere Teilchengröße (ASTM C690) 3 µm Teilchengröße D50 (ISO 13320-1) 4,5µm [4] spez. Oberfläche 110 m²/g, Primärteilchengröße durchschnittlich 16 nm [5] spez. Oberfläche 220 m²/g, Primärteilchengröße durchschnittlich 7 nm [6] spez. Oberfläche 50 m²/g, Primärteilchengröße durchschnittlich 40 nm [7] spez. Oberfläche 185 m²/g [8] spez. Oberfläche 475 m²/g, Mittlere Teilchengröße (ASTM C690) 4,5 µm Teilchengröße D50 (ISO 13320-1) 6µm | | | | | |

Die wichtigsten Beurteilungskriterien sind Transparenz, Gelbton (b-Wert), mechanische Eigenschaften und Konsistenz. Werte unter 70 % in der Transparenz wurden negativ gewertet (hier wird ein gering gefülltes Material betrachtet, hochgefüllte Komposite mit ca. 70 - 75 Gew. % Füllstoffanteil erreichen typischerweise mind. ca. 60 %).

Zu hohe Gelbwerte (b-Wert > 20) wurden ebenfalls ausgeschlossen.

Eine wichtige Eigenschaft eines Rheologiemodifizierers ist ein gewisser Verdickungseffekt. Deshalb wurden Rohstoffe mit einer Viskositätsbewertung <= 3 (Skala von 1 - 6) ebenfalls negativ bewertet.

Somit ergibt sich aus dieser Versuchsreihe für die beiden Produkte aus der Nanogel-Reihe die größtmögliche Transparenz bei geringstem Gelbwert und höchstmöglicher Verdickungswirkung.

| Tabelle II | **b-Wert** | **Transparenz** | **Biegefestigkeit** | **E-Modul** |
|---|---|---|---|---|
| **Rheologie- Modifizierer** | | **[%]** | **[MPa]** | **[MPa]** |
| Aerosil 380 | 13,2 | 65,8 | 89 | 5009 |
| Sipernat 160 [9] | 12,0 | 67,5 | 81 | 4660 |
| Aerosil 130 | 11,2 | 66,2 | 76 | 4178 |
| Sipernat LS 500 | 12,0 | 67,3 | 90 | 4406 |
| Sipernat 570 | 12,6 | 65,0 | 81 | 4453 |
| Aerosil R812s | 11,4 | 68,8 | 75 | 3881 |
| Aerosil R972 | 10,8 | 66,7 | 81 | 4367 |
| Dendrimer PU | 11,2 | 63,6 | 80 | 3710 |
| Nanogel TLD 201 [10] | 10,6 | 65,5 | 83 | 3882 |

| | | | | |
|---|---|---|---|---|
| [9] spez. Oberfläche 165 m²/g, Mittlere Teilchengröße (ASTM C690) 7 µm Teilchengröße D50 (ISO 13320-1) 11 µm [10] Spez. Oberfläche 700-800 m²/g Teilchengröße 7-11 µm | | | | |

Pasten mit jeweils gleich hoher Konzentration an Rheologiemodifizierer (Nanogel aufgrund der extrem verdickenden Wirkung nur 2 Gew.%); die meisten Produkte weisen einen zu hohen Gelbwert auf.

Anpassung der Konzentration des Rheologiemodifizierers auf identische Handlingseigenschaften ergibt folgende Produkteigenschaften:

| Tabelle III | **Rheologie- Modifizierer** | **b-Wert** | **Transparenz** | **Biegefestigkeit [MPa]** | **E-Modul [MPa]** |
|---|---|---|---|---|---|
| **VP** | | | | | |
| HOK4A_4% | Sipernat LS 500 | 11,41 | 67,18 | 89 | 4367 |
| HOK9A_2,5 % | Nanogel TLD 201 | 10,98 | 66,02 | 93 | 4308 |
| HOK7A_2% | Aerosil R972 | 10,66 | 66,38 | 83 | 4423 |
| HOK1A_3% | Aerosil 380 | 10,84 | 66,46 | 88 | 4442 |

Hier zeigt die Paste mit dem Rheologiemodifizierer Nanogel TLD 201 die ausgewogensten Produkteigenschaften:
- geringer b-Wert (< 11)
- hohe Transparenz (< 60 %)
- hohe Biegefestigkeit bei gleichzeitig geringerem E-Modul (elastisches Verhalten, ideal für Verblendkomposite)

### Vergleich mit agglomerierter Kieselsäure

### Einer Kompositrezeptur

| **Monomere** | Gew.Teile |
|---|---|
| Bis-GMA | 25,0 |
| Difunktionelles aliphatisches Urethanacrylat | 10,0 |
| HPMA, 2-Hydroxypropylmethacrylat | 10,0 |

| **Stabilisatoren/Initiatoren/Akzeleratoren** | |
|---|---|
| 2, 6-Di-tert-butyl-4-cresol | 0,02 |
| DL-Campherchinon | 0,1 |
| Aminfunktionelles Acrylat | 0,2 |
| 2,2-Dimethoxy-1,2-diphenylethan-1-one, | 0,08 |
| Irgacure 651 (Ciba Specialty Chemicals) | |

| **Glasfüller** | |
|---|---|
| Bariumaluminiumsilikatglas ∼ 1µm - silanisiert | 50 |

Es wurden 3 verschiedene feinteilige Füllstoffe zugesetzt und die mechanischen und Farbeigenschaften bestimmt:

| | | | | |
|---|---|---|---|---|
| **Feinteiliger Füllstoff** | Nanogel TLD 201 | 4,6 | | |
| | Nanogel 620 | | 4,6 | |
| | Aerosil R972 | | | 4,6 |
| **3-Punkt Biegeversuch** | Biegefestigkeit | 127,4 | 140 | 127,7 |
| | E-Modul | 6042 | 6065 | 5995 |
| **Farbwerte** | L* | 90,55 | 90,78 | 91,03 |
| | a* | -1,82 | -2 | -1,89 |
| | b* | 7,92 | 7,03 | 7,04 |
| | C* | 8,13 | 7,31 | 7,29 |
| | h | 102,96 | 105,88 | 105,06 |
| | Transparenz [%] | 79,78 | 79,5 | 73,3 |

## Patentansprüche

1. Verwendung von Kieselsäuren zur Einstellung der Gebrauchseigenschaften von Dentalmaterial, **dadurch gekennzeichnet, dass** die Kieselsäure hochdispers ist, einen Porendurchmesser von 15-30 nm, eine innere Porenstruktur, eine Korngröße von d₅₀ 7 - 10 µm und eine hydrophobe Oberflächenmodifizierung durch Oberflächenbelegung mit Trimethylsilylgruppen aufweist, und die Kieselsäure als Rheologiemodifizierer in einem Verblendkomposit oder einem Füllungskomposit oder einem Prothesenwerkstoff oder einem Material zur Herstellung künstlicher Zähne oder einem Dentalzement oder einem Abformmaterial oder einem Pastenopaker eingesetzt wird.

2. Verwendung nach Anspruch 1, wobei die Kieselsäure aufgrund ihrer Porosität zu 95% aus Luft besteht.

3. Verwendung nach Anspruch 1 oder 2 **dadurch gekennzeichnet, dass** die hochdisperse Kieselsäure eine spezifische Oberfläche von > 700 m²/g aufweist.

4. Verwendung nach einem der vorstehenden Ansprüche, **dadurch gekennzeichnet, dass** die Kieselsäure hydrophobiert ist.

5. Verwendung nach einem der vorstehenden Ansprüche, wobei die Kieselsäure eine durchschnittliche Primärpartikelgröße von < 100 nm besitzt.

6. Verwendung nach einem der vorstehenden Ansprüche, **dadurch gekennzeichnet, dass** die hochdisperse Kieselsäure aus agglomerierten Primärpartikeln mit einem überwiegenden Partikeldurchmesser von 20 - 50 nm besteht.

7. Verwendung nach einem der vorstehenden Ansprüche, **dadurch gekennzeichnet, dass** die hochdisperse Kieselsäure aus agglomerierten Primärpartikeln mit einem überwiegenden Partikeldurchmesser von 20 - 40 nm besteht.

8. Verwendung nach einem der vorstehenden Ansprüche, **dadurch gekennzeichnet, dass** die agglomerierten Partikel der hochdispersen Kieselsäure durch hohe Scherkräfte, insbesondere Drei-Walzenstuhl, in die Primärpartikel aufgespalten werden.

9. Verfahren zur Einstellung der Gebrauchseigenschaften von Dentalmaterial
mit den Schritten:
**A** Herstellen einer Vor-Mischung aus mindestens Dentalmonomer(en), Initiator(en) und Kieselsäure mit innerer Porenstruktur mit Porendurchmesser von 15 - 30 nm und einer hydrophoben Oberflächenmodifizierung durch Oberflächenbelegung mit Trimethylsilylgruppen;
**B** Behandeln der Vormischung mit einem Mischgerät,
**C** Nachbehandeln der entstanden Mischung mit starken Scherkräften.

10. Verfahren nach Anspruch 9, wobei Schritt **B** mit einem Rotationsmischgerät durchgeführt wird.

11. Verfahren nach Anspruch 9, wobei Schritt **C** mit einem Dreiwalzenstuhl, einem Rotor-Stator-Mischer oder Ultraschalleinwirkung durchgeführt wird.

## Claims

1. Use of silicic acids for adjustment of the performance characteristics of dental material, **characterised in that** the silicic acid is highly dispersed, has a pore diameter of 15 - 30 mm, an inner pore structure, a grain size of d₅₀ 7 - 10 µm and a hydrophobic surface modification by surface coating with trimethylsilyl groups, and the silicic acid is used as rheology modifier in a veneering composite or a filling composite or a prosthesis material or a material for the production of artificial teeth or a dental cement or an impression material or a paste opaque.

2. Use according to claim 1, wherein the silicic acid consists of 95% air due to its porosity.

3. Use according to claim 1 or 2, **characterised in that** the highly dispersed silicic acid has a specific surface of > 700 m²/g.

4. Use according to any one of the preceding claims, **characterised in that** the silicic acid is hydrophobised.

5. Use according to any one of the preceding claims, wherein the silicic acid has an average primary particle size of < 100 nm.

6. Use according to any one of the preceding claims, **characterised in that** the highly dispersed silicic acid consists of agglomerated primary particles having a predominant particle diameter of 20 - 50 nm.

7. Use according to any one of the preceding claims, **characterised in that** the highly dispersed silicic acid consists of agglomerated primary particles having a predominant particle diameter of 20 - 40 nm.

8. Use according to any one of the preceding claims, **characterised in that** the agglomerated particles of the highly dispersed silicic acid are cleaved into the primary particles by high shear forces, in particular by a three roll mill.

9. Method for adjustment of the performance characteristics of dental material with the steps:
A producing a pre-mixture of at least dental monomer(s), initiator(s) and silicic acid having an inner pore structure having a pore diameter of 15 - 30 nm and a hydrophobic surface modification by surface coating with trimethylsilyl groups;
B treating the pre-mixture with a mixer,
C post-treating the resulted mixture with strong shear forces.

10. Method according to claim 9, wherein step B is performed using a rotary mixer.

11. Method according to claim 9, wherein step C is performed using a triple roll mill, a rotor-stator mixer or ultrasound treatment.

## Revendications

1. Utilisation des acides siliciques pour l'ajustage des caractéristiques de performances d'une matière dentaire, **caractérisée en ce que** l'acide silicique est hautement dispersé, a un diamètre des pores de 15 - 30 nm, une structure de pores interne, une granulométrie de d₅₀ 7 - 10 µm et une modification de surface hydrophobe par un revêtement de surface avec des groupes triméthylsilyles, et l'acide silicique est utilisé en tant qu'un modificateur de rhéologie dans un composite obturant ou un composite facettant ou une matière de prothèse ou une matière pour la production des dents artificielles ou un ciment dentaire ou une matière d'empreinte ou une pâte d'opaque.

2. Utilisation selon la revendication 1, selon laquelle l'acide silicique se compose à 95% d'air en raison de sa porosité.

3. Utilisation selon la revendication 1 ou 2, **caractérisée en ce que** l'acide silicique hautement dispersé a une surface spécifique de > 700 m²/g.

4. Utilisation selon l'une des revendications précédentes, **caractérisée en ce que** l'acide silicique est hydrophobisé.

5. Utilisation selon l'une des revendications précédentes, dans laquelle l'acide silicique a une taille moyenne de particules primaires de < 100 nm.

6. Utilisation selon l'une des revendications précédentes, **caractérisée en ce que** l'acide silicique hautement dispersé se compose des particules primaires agglomérées ayant un diamètre prédominant de particules de 20 - 50 nm.

7. Utilisation selon l'une des revendications précédentes, **caractérisée en ce que** l'acide silicique hautement dispersé se compose des particules primaires agglomérées ayant un diamètre prédominant de particules de 20 - 40 nm.

8. Utilisation selon l'une des revendications précédentes, **caractérisée en ce que** les particules agglomérées de l'acide silicique hautement dispersé sont clivées en les particules primaires par des forces de cisaillement élevées, en particulier par un moulin à trois rouleaux.

9. Procédé pour l'ajustement des caractéristiques de performances d'une matière dentaire avec les étapes :
A produisant un prémélange d'au moins des monomère(s) dentaire(s), des initiateur(s) et l'acide silicique ayant une structure de pores interne ayant un diamètre de pores de 15 - 30 nm et une modification de surface hydrophobe par un revêtement de surface avec des groupes triméthylsilyles ;
B traitant le prémélange avec un mélangeur,
C post-traitant le mélange résulté avec des forces de cisaillement fortes.

10. Procédé selon la revendication 9, dans lequel l'étape B est effectuée utilisant un mélangeur rotatif.

11. Procédé selon la revendication 9, dans lequel l'étape C est effectuée utilisant un moulin à trois rouleaux, un mélangeur rotor-stator ou un traitement par ultrasons.
